# EUROPEAN PATENT SPECIFICATION

(11) **EP 3 672 640 B1**
(45) Date of publication and mention of the grant of the patent: **20.11.2024**
(21) Application number: 18891625.8
(22) Date of filing: 15.08.2018
(51) Int. Cl.: A61K 47/36, A61K 9/50, A61K 35/741, A01N 25/28, A23L 3/3571, A61K 9/16, C12N 1/04, C12N 1/20, A23L 3/3463, A61K 8/11, A61K 8/362, A61K 8/365, A61K 8/73, A61K 8/9728, A61Q 19/00, A61K 8/99

(54) **MICROCAPSULES LOADED WITH PROBIOTICS AND PRODUCTION THEREOF**
MIT PROBIOTIKA BELADENE MIKROKAPSELN UND IHRE HERSTELLUNG
MICROCAPSULES CHARGÉES DE PROBIOTIQUES ET LEUR PRODUCTION

(30) Priority: 21.08.2017 TR 201712406
(43) Date of publication of application: 01.07.2020
(73) Proprietor: Nanomik Biyoteknoloji A.S., Sariyer/Istanbul (TR)
(72) Inventor: BERBER, Buse, Kagithane Istanbul (TR)
(74) Representative: Kaya, Erdem
(86) International application number: PCT/TR2018/050434
(87) International publication number: WO 2019/125332

(56) References cited:
- CN-A- 101 319 210
- CN-A- 101 319 210
- KR-A- 20090 100 033
- KR-A- 20090 100 033
- KR-A- 20100 092 923
- KR-A- 20100 092 923
- LILIANA CAETANO ET AL: "Effect of Experimental Parameters on Alginate/Chitosan Microparticles for BCG Encapsulation", MARINE DRUGS, vol. 14, no. 5, 11 May 2016 (2016-05-11), pages 90, XP055663816, DOI: 10.3390/md14050090
- SU HUNG CHING ET AL: "Alginate gel particles-A review of production techniques and physical properties", CRITICAL REVIEWS IN FOOD SCIENCE AND NUTRITION, vol. 57, no. 6, 15 May 2015 (2015-05-15), USA, pages 1133 - 1152, XP055463673, ISSN: 1040-8398, DOI: 10.1080/10408398.2014.965773
- SARMENTO ET AL: "Insulin-Loaded Nanoparticles are Prepared by Alginate Ionotropic Pre-Gelation Followed by Chitosan Polyelectrolyte Complexation", JOURNAL OF NANOSCIENCE AND NANOTECHNOLOGY, AMERICAN SCIENTIFIC PUBLISHERS, US, vol. 7, no. 8, August 2007 (2007-08-01), pages 2833 - 2841, XP009520611, ISSN: 1533-4880, DOI: 10.1166/JNN.2007.609
- SUKMAWATI ANITA ET AL: "Effect of tween 80 on nanoparticle preparation of modified chitosan for targeted delivery of combination doxorubicin and curcumin analogue", IOP CONFERENCE SERIES: MATERIALS SCIENCE AND ENGINEERING, vol. 311, February 2018 (2018-02-01), GB, pages 012024, XP055783356, ISSN: 1757-8981, Retrieved from the Internet <URL:http://stacks.iop.org/1757-899X/311/i=1/a=012024?key=crossref.2eb0a097fe00e4746d775e9db50ea762> DOI: 10.1088/1757-899X/311/1/012024
- MICHAEL T. COOK ET AL: "Production and Evaluation of Dry Alginate-Chitosan Microcapsules as an Enteric Delivery Vehicle for Probiotic Bacteria", BIOMACROMOLECULES, vol. 12, no. 7, 11 July 2011 (2011-07-11), US, pages 2834 - 2840, XP055420206, ISSN: 1525-7797, DOI: 10.1021/bm200576h
- CHAVARRI M ET AL: "Microencapsulation of a probiotic and prebiotic in alginate-chitosan capsules improves survival in simulated gastro-intestinal conditions", INTERNATIONAL JOURNAL OF FOOD MICROBIOLOGY, ELSEVIER BV, NL, vol. 142, no. 1-2, 15 August 2010 (2010-08-15), pages 185 - 189, XP027196657, ISSN: 0168-1605, [retrieved on 20100630]
- COOK, MICHAEL T. ET AL.: "Production and evaluation of dry alginate-chitosan microcapsules as an enteric delivery vehicle for probiotic bacteria", BIOMACROMOLECULES, vol. 12.7, 2011, pages 2834 - 2840, XP055420206, doi:10.1021/bm200576h
- CHAVARRI, MARIA ET AL.: "Microencapsulation of a probiotic and prebiotic in alginate-chitosan capsules improves survival in simulated gastro-intestinal conditions", INTERNATIONAL JOURNAL OF FOOD MICROBIOLOGY, vol. 142, no. 1-2, 2010, pages 185 - 189, XP027196657

## Description

### Technical Field

The invention is related to the production method of the microencapsulated form of probiotics with the chitosan-alginate polymers, the microcapsules loaded with probiotics obtained by this method and their use in food, agriculture and cosmetics.

### Known State of the Art (Prior Art)

Mycotoxins are toxic metabolic products produced by some moulds such as *Aspergillus, Penicillium* and *Fusarium,* which can be found in foods and agricultural products generally as a result of contamination. Mycotoxins being formed during the cultivation, storage, processing or transportation of foods cause varying degrees of toxicosis (mycotoxicosis) in humans and/or animals. Preventing the growth of mould in foods is difficult, however, the amount of these can be minimized during the processing and storage of foods by providing hygienic conditions. Forming of moulds which produce toxins is an exceptional problem in the developing countries, that is because in these countries, there are no controlled storage conditions such as in the developed countries. Besides, in tropical zones having warm and humid climate, the risk of mould growth in food increases.

After the mycotoxin is formed, mycotoxins can be removed from the food products by binders. However, since this method is used after mycotoxin formation, it deteriorates the quality of the product, causes changes in taste and leads to chemical pollution. Also, since most of the binders used today are mycotoxin-specific, they do not present sufficient efficiency with products contaminated with more than one mycotoxin.

Due to reasons as such, the use of microorganisms which support consumer health and which have immune system-stimulating effects in the production of food and agricultural products increases. It is known that the probiotics which constitute a significant group of these microorganisms play an important role in the prevention and treatment of gastrointestinal system disorders and formation of normal microflora. Use of probiotics in food stuff is longstanding. However, addition of probiotics directly into the product causes change in taste and quality.

Also, it is known that the probiotic technology boosts the immune system, repairs the natural defence mechanism, prevents collagen structure damage and slows the aging process by providing the water balance of the skin.

When the inventions similar to the subject invention are analysed, the following documents are found:
- US20060008511A1:In this patent document, a preparation, which comprises a probiotic encapsulated with a mix of xanthan gum and chitosan gum as ingredients, developed for pets, is described.
- US20120128821A1:In this patent document, a method related to isolating probiotic from donkey's milk is described.

KR20090100033A, KR20100092923A, CN101319210A, Cook M T et al, Biomacromolecules, vol. 12, no. 7, 2011-07-11, pages 2834-2840 and Chavarri M et al, International Journal of Food Microbiology, vol. 142, no. 1-2, 2010-08-15, pages 185-189 all disclose the preparation of microcapsules comprising probiotics from alginate solutions comprising 1-3wt% alginate.

Caetano L et al, Marine Drugs, vol. 14, no. 90, 2016-05-11, page 1-30; Su Hung Ching et al, Critical Reviews in Food Science and Nutrition, vol. 57, no. 6, 2015-05-15, pages 1133-1152 and Sarmento et al, Journal of Nanoscience and Nanotechnology, vol. 7, no. 8, 2007-08, pages 2833-2841 disclose the preparation of microcapsules from very dilute alginate solutions and chitosan comprising entrapped agents. The entrapment of probiotics is not disclosed.

### Brief Description and Purposes of the Invention

The invention is related to microcapsules obtained by microencapsulating endospore form of probiotics with chitosan-alginate polymers. The microcapsules loaded with said probiotics prevent mycotoxigenic moulds and the mycotoxins which the moulds produce through biological struggle. In this study, encapsulation of probiotics is carried out especially in order to prevent probiotics from being damaged by gastric acid or in order to increase the quality in fermented products.

The microcapsules loaded with probiotics related to the subject invention;
- Prevent the contamination of mycotoxin to food products at the rate of 95% with different combinations of probiotics,
- Do not cause any alteration in the colour and appearance of the product they are applied to,
- Affect many mycotoxin species of different types since they suppress the mould growth and stress mechanism in food and agricultural products,
- Have antifungal effect,
- Do not necessitate extra disinfection since at the same time, they will also have an antifungal effect on the product they are being applied to,
- Are natural and harmless,
- Provide protection in storage up to 360 days by extending the shelf life of the product they are applied to.

### Detailed Description of the Invention

The present invention is related to chitosan-alginate microcapsules loaded with probiotics which present antifungal and anti-mycotoxin activity in fields such as food, agriculture, cosmetics and healthcare.

The production method of the microcapsules loaded with the probiotic according to the invention contains the procedure steps of;
- Dissolving the alginate in water which is between 0,002- 0,1% ratio by weight,
- Autoclaving the alginate solution at 110-130C°temperature, preferably at 121C° and then sterilizing the same,
- Mixing the probiotics with alginate,
- Adding calcium chloride (CaCl₂) between 0,01-1 M, preferably 0,1 M to the probiotic-alginate mix and stirring,
- Dissolving chitosan in 0,25-3% concentration by weight in 0.5-5% organic acid by volume(lactic acid, acetic acid, ascorbic acid, citric acid, tartaric acid, malic acid etc.), preferably in 1% organic acid (In this stage, the pH is adjusted between 3.5-7).
- Autoclaving the chitosan solution at 110-130°C temperature, preferably at 121°C and then sterilizing the same,
- Thereafter, combining the 3:1 chitosan solution and the alginate-calcium chloride (CaCl₂) solution containing the probiotics in a container dropwise,
- Adding tween 80 (Polysorbate 80) between 0.01-2% by volume and stirring the mixture until homogenization is obtained.

The probiotics used in the invention are chosen from the microorganisms of *Bacillus laterosporus, Bacillus sphaericus, Bacillus subtilis, Bacillus coagulans, Streptococcus thermophilis, Azotobacter, Azospirillum, Agrobacterium, Gluconobacter, Flavobacterium, Herbaspirillum, Bacillus megaterium, Thiobacillus,* , *B. polymyxa, B. brevis, B. licheniformis, B. circulans, B. cereus, B. thuringiensis, B. longum, B. breve, B. infantis, L. helveticus, L. rhamnosus, L. plantarum, L. casei* , *L. acidophilus* , *Lactobacillus delbreckii, Lactobacillus ellobiosus, Lactobacillus lactis, Lactobacillu cidophilus, Lactobacillus reuteri, Lactobacillus brevis, Lactobacillus casei, Lactobacillus curvatus, Lactobacillus fermentum, Lactobacillus plantarum, Lactobacillus helveticus, Streptococcus cremoris, Streptococcus thermophiles, Streptococcus intemedius, Streptococcus lactis, Streptococcus diacetilactis, Enterococcus feacalis, Lactococcus spp, Lactococcus lactis subsp., Pediococcus spp., Pediococcus cerevisiae, Pediococcus acidilactici, Pediococcus pentosaceus, Bifidobacterium spp. Bifidobacterium animalis, Bifidobacterium adolecentis, Bifidobacterium bifidum, Bifidobacterium breve, Bifidobacterium infantis, Bifidobacterium longum, Leuconostoc spp. L. salivarius, L. paracasei, L. gasseri, L. reuteri, B. Bifidum, B. longum, B. infantis, Lb. delbrueckii, Lb. plantarum, Lb. pentosus, Lb. brevis, P. damnosus, Lb. collinoides, Lb. pentosus, Pediococcus spp., Lb. buohneri, Leuconostoc mesenteroides, Pedococcus pentosaceus, Lb. casei, Lb. kefir, Lb. acidophilus, Lb. helveticus, Lb. casei, Lb. bulgaricus, Lb. lactis, Lb. plantarum, Lb. brevis* , *Acetobacter spp., Streptococcus genera, Streptococcus spp., Streptococcus lactis, S. thermophilus, S. durans, S. cremoris, Lactobacillus alimentarus, L. Alimentarus, L. multaromicus, L. sanfrancisco, Lactococcus lactis ssp, S. cerevisia, Lactobacillus sakei, Lactobacillus alimentarius, Lactobacillus paralimentarius, Lactobacillus paracasei, Lactobacillus buchneri, Enterococcus faecium, Enterococcus mundtii, Enterococcus faecelis, Enterococcus casseliflavus, Lactobacillus pentosus, Enterococcus faecium, Pediococcus pentosaceus, Lactobacillus farciminis, Pichia kudriavzevii, Lactobacillus farciminis, Lactobacillus casei, Lactobacillus alimentarius, Pichia kudriavzevii* , *Candida humilis, L.lactis subsp. cremoris, Lb. delbrueckii subsp. lactis, Lb.helveticus, Lb.casei, Lb. delbrueckii subsp. , Leuc.mesenteroides subsp. cremoris, Lb. johsonii, Lb.kefirenofacies, Lb.curvatus, P.acidiiactici, P.pentosaceus, Lb. alimentarius, C. piscicola, Leuc.mesenteroides, Pacidilactici, P. cere visiae, Lb.pentosus, P.acidilactici, Thalophilus, Lb.sanfransiscensis, Lb.farciminis, Lb.fermentum, Lb.amylovorus, Lb. reuteri, Lb.pontis, Lb.panis, Lb.alimentarius, W. cibaria, O.oeni, L.coryniformis, L. curvatus, L.jugurti, L.jensenii, L.bucheneri, L.cellobiosus, L.coprophilus, L.hilgardii, L.leichmannii, L. dextranicum, Pacidiiactici, P.pentosaceus, S. thermophilus, L.lactis subsp. diacety lactis, L.lactis subsp. hordniae, L.garvieae, L.rafinolactis, V. flu vialis, V.salmoninarum, Leuconostoc sp., L. cremoris, L. dextranicum, L.mesenteroides, L.paramesenteroides, L.gelidum, L.carnosum, Carnobacterium sp., C.divergens, C.mobile, Cgallinarum, C.piscicola, Vagococcus sp., V.fluvialis, V.salmoninarum, L.garvieae, Lactococcus diacetylactis, Propionibacterium freudenreichii, Pediococcus sp, S.uvarum, Lb.coryniformis, Candida crusei, Weissella confusa, Hansenula silvicola, Debaryomyces hansenii, Trichosporon beigelli, Bacillus amyloliquefaciens, Torulopsis sp., Candida mycoderma, Lb.buchneri, Pacidiiactici, Pediococcus pentasaceus, L.cellobiosus, E.mundtii*/*E.gallinarum, Ecasseliflavus, Purinae-equi, Lb.murinus, Candida milleri, Eburtonii, Efibulinger, Issatchenkia orientalis, Candida pelliculosa, C.tropicalis, Pediococcus acidilactici, Pseudoplantarum, Pediococcus acidilactici, Pediococcus pentosaceus, Leuconostoc pseudomesenteroides, Weissella cibaria, Lb. paraplantarum, Issatchenkia orientalis, Candida glabrata, Pediococcus acidilactici, Kluyveromyces marxianus, Pichia kudriavzevii, Saccharomyces servazzi, Torulaspora delbrueckii, Kazachstania unispora, Saccharomyces barnettii.*

The application of the product related to the invention to food and agricultural products is as follows:
- 10 ml of the solution containing 10⁹ cfu bacteria is mixed with 1 L water in order to provide protection against mycotoxin in the garden or during the storage period,
- The same is applied on the products by means of a spraying method.

## Claims

1. Method of producing polymeric microcapsules containing a probiotic which is protective against mould and mycotoxin formation, **characterized in that** it comprises the procedure steps of;
- Dissolving the alginate in water which is between 0,002- 0,1% ratio by weight,
- Autoclaving the alginate solution at 110-130°C temperature, preferably at 121°C and then sterilizing the same,
- Mixing the probiotics with alginate,
- Adding calcium chloride (CaCl₂) between 0,01-1 M, preferably 0,1 M to the probiotic-alginate mix and stirring,
- Dissolving chitosan in 0,25-3% concentration by weight in 0.5-5% organic acid by volume,
- Autoclaving chitosan solution at 110-130°C temperature, and then sterilizing the same,
- Thereafter, combining the 3:1 chitosan solution and the alginate-calcium chloride (CaCl₂) solution containing the probiotics in a container dropwise,
- Adding tween 80 (Polysorbate 80) between 0.01-2% by volume and stirring the mix until homogenization is obtained.

2. A method according to Claim 1, **characterized in that** the organic acid is lactic acid, acetic acid, ascorbic acid, citric acid, tartaric acid or malic acid.

3. A method according to Claim 1, **characterized in that** the pH is adjusted to be between 3.5-7 in the procedure step in which the chitosan is dissolved in organic acid.

4. A method according to Claim 1, **characterized in that** the alginate solution is autoclaved at 121°C and then sterilized.

5. A method according to Claim 1, **characterized in that** the chitosan solution is autoclaved at 121°C and then sterilized.

6. A method according to Claim 1, **characterized in that** 0,1 M calcium chloride (CaCl₂) is added to the probiotic-alginate mix and stirred.

7. The method in which 0,25-3% chitosan by weight is dissolved in 1% organic acid.

8. A method according to Claim 1, **characterized in that** said probiotics are bacteria and/or yeast.

9. A method according to Claim 8, **characterized in that** the probiotics are chosen from the microorganisms of *Bacillus laterosporus, Bacillus sphaericus, Bacillus subtilis, Bacillus coagulans, Streptococcus thermophilis, Azotobacter, Azospirillum, Agrobacterium, Gluconobacter, Flavobacterium, Herbaspirillum, Bacillus megaterium, Thiobacillus,* , *B. polymyxa, B. brevis, B. licheniformis, B. circulans, B. cereus, B. thuringiensis, B. longum, B. breve, B. infantis, L. helveticus, L. rhamnosus, L. plantarum, L. casei, L. acidophilus, Lactobacillus delbreckii, Lactobacillus ellobiosus, Lactobacillus lactis, Lactobacillu cidophilus, Lactobacillus reuteri, Lactobacillus brevis, Lactobacillus casei, Lactobacillus curvatus, Lactobacillus fermentum, Lactobacillus plantarum, Lactobacillus helveticus, Streptococcus cremoris, Streptococcus thermophiles, Streptococcus intemedius, Streptococcus lactis, Streptococcus diacetilactis, Enterococcus feacalis, Lactococcus spp,Lactococcus lactis subsp., Pediococcus spp., Pediococcus cerevisiae, Pediococcus acidilactici, Pediococcus pentosaceus, Bifidobacterium spp. Bifidobacterium animalis, Bifidobacterium adolecentis, Bifidobacterium bifidum, Bifidobacterium breve, Bifidobacterium infantis, Bifidobacterium longum, Leuconostoc spp. L. salivarius, L. paracasei, L. gasseri, L. reuteri, B. Bifidum, B. longum, B. infantis, Lb. delbrueckii, Lb. plantarum, Lb. pentosus, Lb. brevis, P. damnosus, Lb. collinoides, Lb. pentosus, Pediococcus spp., Lb. buohneri, Leuconostoc mesenteroides, Pedococcus pentosaceus, Lb. casei, Lb. kefir, Lb. acidophilus, Lb. helveticus, Lb. casei, Lb. bulgaricus, Lb. lactis, Lb. plantarum, Lb. brevis* , *Acetobacter spp., Streptococcus genera, Streptococcus spp., Streptococcus lactis, S. thermophilus, S. durans, S. cremoris, Lactobacillus alimentarus, L. Alimentarus, L. multaromicus, L. sanfrancisco, Lactococcus lactis ssp, S. cerevisia, Lactobacillus sakei, Lactobacillus alimentarius, Lactobacillus paralimentarius, Lactobacillus paracasei, Lactobacillus buchneri, Enterococcus faecium, Enterococcus mundtii, Enterococcus faecelis, Enterococcus casseliflavus, Lactobacillus pentosus, Enterococcus faecium, Pediococcus pentosaceus, Lactobacillus farciminis, Pichia kudriavzevii, Lactobacillus farciminis, Lactobacillus casei, Lactobacillus alimentarius, Pichia kudriavzevii, Candida humilis, L.lactis subsp. cremoris, Lb.delbrueckii subsp. lactis, Lb. helveticus, Lb.casei, Lb. delbrueckii subsp.* , *Leuc.mesenteroides subsp. cremoris, Lb. johsonii, Lb.kefirenofacies, Lb. curvatus, P.acidilactici, P. pentosaceus, Lb. alimentarius, C.piscicola, Leuc.mesenteroides, P. acidilactici, P.cerevisiae, Lb.pentosus, P. acidilactici, T.halophilus, Lb.sanfransiscensis, Lb.farciminis, Lb.fermentum, Lb.amylovorus, Lb.reuteri, Lb.pontis, Lb. panis, Lb.alimentarius, W.cibaria, O.oeni, L.coryniformis, L. curvatus, L jugurti, L.jensenii, L.buclieneri, L.cellobiosus, L.coprophilus, L.hilgardii, L.leichmannii, L.dextranicum, P.acidilactici, P. pentosaceus, S.thermophilus, L.lactis subsp. diacety lactis, L.lactis subsp. hordniae, L.garvieae, L.rafiηolactis, V.fluvialis, V. salmoninarum, Leuconostoc sp., L.cremoris, L.dextranicum, L.mesenteroides, L. paramesenteroides, L.gelidum, L.carnosum, Carnobacterium sp., C. divergens, C.mobile, C.gallinarum, Cpiscicola, Vagococcus sp., V. fluvial is, V.salmoninarum, L.garvieae, Lactococcus diacetylactis, Propionibacterium freudenreichii, Pediococcus sp, S.uvarum, Lb.coryniformis, Candida crusei, Weissella confusa, Hansenula silvicola, Debaryomyces hansenii, Trichosporon beigelli, Bacillus amyloliquefaciens, Torulopsis sp., Candida mycoderma, Lb.buchneri, P. acidilactici, Pediococcus pentasaceus, L.cellobiosus, E.mundtii*/*E.gallinarum, E.casseliflavus, P.urinae-equi, Lb.murinus, Candida milleri, E.burtonii, E.fibulinger, Issatchenkia orientalis, Candida pelliculosa, C. tropicalis, Pediococcus acidilactici, Pseudoplantarum, Pediococcus acidilactici, Pediococcus pentosaceus, Leuconostoc pseudomesenteroides, Weissella cibaria, Lb. paraplantarum, Issatchenkia orientalis, Candida glabrata, Pediococcus acidilactici, Kluyveromyces marxianu, Pichia kudriavzevi, Saccharomyces servazzi, Torulaspora delbrueckii, Kazachstania unispora, Saccharomyces barnettii.*

10. Microcapsule loaded with the probiotic which is protective against mould and mycotoxin formation, produced by the method according to Claim 1.

11. Use of the microcapsule loaded with probiotics according to Claim 10 in agriculture.

12. Use of the microcapsule loaded with probiotics according to Claim 11 with grape, corn, almond, hazelnut, peanut derivatives, walnut, fresh/dry fruits/vegetables or grain products.

13. Use of the microcapsule loaded with probiotics according to Claim 10 with stored agricultural products.

14. Use of the microcapsule loaded with probiotics according to Claim 10 with food products selected from cheese, olives, yoghurt, meat products, fruits and vegetables or cosmetic products selected from toiletry, creams, gels and emulsions

## Patentansprüche

1. Verfahren zur Herstellung von polymeren Mikrokapseln, die ein Probiotikum enthalten, das gegen Schimmel- und Mykotoxinbildung schützt, **dadurch gekennzeichnet, dass** es die folgenden Verfahrensschritte umfasst:
- Auflösen des Alginats in Wasser, das zwischen 0,002-0,1 Gew.-% liegt,
- Autoklavieren der Alginatlösung bei einer Temperatur von 110-130°C, vorzugsweise bei 121°C, und anschließendes Sterilisieren derselben,
- Mischen der Probiotika mit Alginat,
- Zugeben des Calciumchlorids (CaCl₂) zwischen 0,01-1 M, vorzugsweise 0,1 M, zu der Probiotika-Alginat-Mischung und Rühren,
- Auflösen des Chitosans in einer Konzentration von 0,25-3 Gew.-% in 0,5-5 Vol.-% organischer Säure,
- Autoklavieren der Chitosanlösung bei einer Temperatur von 110-130°C und anschließendes Sterilisieren derselben,
- Danach, Kombinieren der 3:1 Chitosanlösung und der Alginat-Calciumchlorid (CaCl₂)-Lösung, die die Probiotika enthält, in einem Behälter tropfenweise,
- Zugeben des Tween 80 (Polysorbat 80) zwischen 0,01-2 Vol.-% und Rühren der Mischung, bis eine Homogenisierung erreicht ist.

2. Verfahren nach Anspruch 1, **dadurch gekennzeichnet, dass** die organische Säure Milchsäure, Essigsäure, Ascorbinsäure, Zitronensäure, Weinsäure oder Apfelsäure ist.

3. Verfahren nach Anspruch 1, **dadurch gekennzeichnet, dass** der pH-Wert in dem Verfahrensschritt, in dem das Chitosan in organischer Säure gelöst wird, auf einen Wert zwischen 3,5 - 7 eingestellt wird.

4. Verfahren nach Anspruch 1, **dadurch gekennzeichnet, dass** die Alginatlösung bei 121°C autoklaviert und anschließend sterilisiert wird.

5. Verfahren nach Anspruch 1, **dadurch gekennzeichnet, dass** die Chitosanlösung bei 121°C autoklaviert und anschließend sterilisiert wird.

6. Verfahren nach Anspruch 1, **dadurch gekennzeichnet, dass** 0,1 M Calciumchlorid (CaCl₂) zu der Probiotik-Alginat-Mischung gegeben und gerührt wird.

7. Verfahren, bei dem 0,25-3 Gew.-% Chitosan in 1 % organischer Säure gelöst werden.

8. Verfahren nach Anspruch 1, **dadurch gekennzeichnet, dass** die Probiotika Bakterien und/oder Hefen sind.

9. Verfahren nach Anspruch 8, **dadurch gekennzeichnet, dass** die Probiotika ausgewählt sind aus den Mikroorganismen von
*Bacillus laterosporus, Bacillus sphaericus, Bacillus subtilis, Bacillus coagulans, Streptococcus thermophils, Azotobacter, Azospirillum, Agrobacterium, Gluconobacter, Flavobacterium, Herbaspirillum, Bacillus megaterium, Thiobacillus,* , *B. polymyxa, B. brevis, B. licheniformis, B. circulans, B. cereus, B. thuringiensis, B. longum, B. breve, B. infantis, L. helveticus, L. rhamnosus, L. plantarum, L. casei, L acidophilus, Lactobacillus delbreckii, Lactobacillus ellobiosus, Lactobacillus lactis, Lactobacillu cidophilus, Lactobacillus reuteri, Lactobacillus brevis, Lactobacillus casei, Lactobacillus curvatus, Lactobacillus fermentum, Lactobacillus plantarum, Lactobacillus helveticus, Streptococcus cremoris, Streptococcus thermophiles, Streptococcus intemedius, Streptococcus lactis, Streptococcus diacetilactis, Enterococcus feacalis, Lactococcus spp, Lactococcus lactis subsp., Pediococcus spp., Pediococcus cerevisiae, Pediococcus acidilactici, Pediococcus pentosaceus, Bifidobacterium spp. Bifidobacterium animalis, Bifidobacterium adolecentis, Bifidobacterium bifidum, Bifidobacterium breve, Bifidobacterium infantis, Bifidobacterium longum, Leuconostoc spp. L salivarius, L. paracasei, L. gasseri, L. reuteri, B. Bifidum, B. longum, B. infantis, Lb. delbrueckii, Lb. plantarum, Lb. pentosus, Lb. brevis, P. damnosus, Lb. collinoides, Lb. pentosus, Pediococcus spp., Lb. buohneri, Leuconostoc mesenteroides, Pedococcus pentosaceus, Lb. casei, Lb. kefir, Lb. acidophilus, Lb. helveticus, Lb. casei, Lb. bulgaricus, Lb. lactis, Lb. plantarum, Lb. brevis, Acetobacter spp., Streptococcus genera, Streptococcus spp., Streptococcus lactis, S. thermophilus, S. durans, S. cremoris, Lactobacillus alimentarus, L. Alimentarus, L multaromicus, L sanfrancisco, Lactococcus lactis ssp,* S. *cerevisia, Lactobacillus sakei, Lactobacillus alimentarius, Lactobacillus paralimentarius, Lactobacillus paracasei, Lactobacillus buchneri, Enterococcus faecium, Enterococcus mundtii, Enterococcus faecelis, Enterococcus casseliflavus, Lactobacillus pentosus, Enterococcus faecium, Pediococcus pentosaceus, Lactobacillus farciminis, Pichia kudriavzevii, Lactobacillus farciminis, Lactobacillus casei, Lactobacillus alimentarius, Pichia kudriavzevii, Candida humilis, L.lactis subsp. cremoris, Lb.delbrueckii subsp. lactis, Lb. helveticus, Lb.casei, Lb.delbrueckii subsp., Leuc.mesenteroides subsp. cremoris, Lb. johsonii, Lb.kefirenofacies, Lb. curvatus, P. acidilactici, P.pentosaceus, Lb. alimentarius, Cpiscicola, Leuc.mesenteroides, P.acidilactici, P.cerevisiae, Lb.pentosus, P.acidilactici, T.halophilus, Lb.sanfransiscensis, Lb.farciminis, Lb.fermentum, Lb.amylovorus, Lb.reuteri, Lb.pontis, Lb.panis, Lb.alimentarius, W. cibaria, O.oeni, Lcoryniformis, Lcurvatus, Ljugurti, Ljensenii, Lbucheneri, Lcellobiosus, Lcoprophilus, Lhilgardii, Lleichmannii, Ldextranicum, P.acidilactici, P.pentosaceus, S. thermophilus, Llactis subsp. diacety lactis, Llactis subsp. hordniae, Lgarvieae, Lrafinolactis, v.fuvialis, V.salmoninarum, Leuconostoc sp., Lcremoris, Ldextranicum, Lmesenteroides, Lparamesenteroides, Lgelidum, Lmmosum, Camobacterium sp., Cdivergens, Cmobile, Cgallinarum, Cpiscicola, Vagococcus sp., v.fuvialis, V.salmoninarum, Lgarvieae, Lactococcus diacetylactis, Propionibacterium freudenreichii, Pediococcus sp, S.uvarum, Lb.coryniformis, Candida crusei, Weissella confusa, Hansenula silvicola, Debaryomyces hansenii, Trichosporon beigelli, Bacillus amyloliquefaciens, Torulopsis sp., Candida mycoderma, Lb.buchneri, P.acidilactici, Pediococcus pentasaceus, Lcellobiosus, Emundtii*/*Egallinarum, E casselifiavus, P.urinae-equi, Lb.murinus, Candida milleri, Eburtonii, E fibulinger, Issatchenkia orientalis, Candida pelliculosa, Ctropicalis, Pediococcus acidilactici, Pseudoplantarum, Pediococcus acidilactici, Pediococcus pentosaceus, Leuconostoc pseudomesenteroides, Weissella cibaria, Lb. paraplantarum, Issatchenkia orientalis, Candida glabrata, Pediococcus acidilactici, Kluyveromyces marxianu, Pichia kudriavzevi, Saccharomyces servazzi, Torulaspora delbrueckii, Kazachstania unispora, Saccharomyces barnettii.*

10. Mikrokapsel, die mit dem Probiotikum beladen ist, das gegen Schimmel und Mykotoxinbildung schützt, hergestellt nach dem Verfahren nach Anspruch 1.

11. Verwendung der mit Probiotika beladenen Mikrokapsel nach Anspruch 10 in der Landwirtschaft.

12. Verwendung der mit Probiotika beladenen Mikrokapsel nach Anspruch 11 mit Trauben-, Mais-, Mandel-, Haselnuss-, Erdnussderivaten, Walnüssen, frischen/trockenen Früchten/Gemüsen oder Getreideprodukten.

13. Verwendung der mit Probiotika beladenen Mikrokapsel nach Anspruch 10 mit gelagerten landwirtschaftlichen Produkten.

14. Verwendung der mit Probiotika beladenen Mikrokapsel nach Anspruch 10 mit Lebensmitteln, ausgewählt aus Käse, Oliven, Joghurt, Fleischprodukten, Obst und Gemüse oder kosmetischen Produkten, ausgewählt aus Toilettenartikeln, Cremes, Gelen und Emulsionen

## Revendications

1. Méthode de production de microcapsules polymères contenant un probiotique qui est protecteur contre la formation de moisissures et de mycotoxines, **caractérisée en ce qu'**elle comprend les étapes de la procédure suivantes;
- Dissolution de l'alginate dans l'eau à un taux compris entre 0,002-0,1 % en poids,
- L'autoclavage de la solution d'alginate à une température de 110-130°C, de préférence à 121°C, et puis la stérilisation de celle-ci,
- Mélange des probiotiques avec de l'alginate,
- Addition de chlorure de calcium (CaCl₂) entre 0,01-1 M, de préférence 0,1 M, au mélange probiotique-alginate et agitation,
- Dissolution du chitosane à une concentration de 0,25-3% en poids dans un acide organique de 0.5-5% en volume,
- Autoclavage d'une solution de chitosane à une température de 110-130°C, et puis la stérilisation de celle-ci,
- Ensuite, la combinaison de la solution de chitosane 3:1 et de la solution d'alginate et de chlorure de calcium (CaCl₂) contenant les probiotiques dans un récipient au goutte-à-goutte,
- Addition de Tween 80 (Polysorbate 80) entre 0.01-2% en volume et agitation du mélange jusqu'à l'obtention d'une homogénéisation.

2. Méthode selon la Revendication 1, **caractérisée en ce que** l'acide organique est l'acide lactique, l'acide acétique, l'acide ascorbique, l'acide citrique, l'acide tartrique ou l'acide malique.

3. Méthode selon la Revendication 1, **caractérisée en ce que** le pH est ajusté pour être compris entre 3.5-7 dans l'étape de la procédure dans laquelle le chitosane est dissous dans l'acide organique.

4. Méthode selon la Revendication 1, **caractérisée en ce que** la solution d'alginate est autoclavée à 121°C et ensuite stérilisée.

5. Méthode selon la Revendication 1, **caractérisée en ce que** la solution de chitosane est autoclavée à 121°C et ensuite stérilisée.

6. Méthode selon la Revendication 1, **caractérisée en ce que** du chlorure de calcium 0,1 M (CaCl₂) est ajouté au mélange probiotique-alginate et agité.

7. Méthode dans laquelle 0,25-3% de chitosane en poids est dissous dans 1% d'acide organique.

8. Méthode selon la Revendication 1, **caractérisée en ce que** lesdits probiotiques sont des bactéries et/ou des levures.

9. Méthode selon la Revendication 8, **caractérisée en ce que** les probiotiques sont choisis parmi les micro-organismes de
*Bacillus laterosporus, Bacillus sphaericus, Bacillus subtilis, Bacillus coagulans, Streptococcus thermophils, Azotobacter, Azospirillum, Agrobacterium, Gluconobacter, Flavobacterium, Herbaspirillum, Bacillus megaterium, Thiobacillus,* , *B. polymyxa, B. brevis, B. licheniformis, B. circulans, B. cereus, B. thuringiensis, B. longum, B. breve, B. infantis, L. helveticus, L. rhamnosus, L. plantarum, L. casei, L acidophilus, Lactobacillus delbreckii, Lactobacillus ellobiosus, Lactobacillus lactis, Lactobacillu cidophilus, Lactobacillus reuteri, Lactobacillus brevis, Lactobacillus casei, Lactobacillus curvatus, Lactobacillus fermentum, Lactobacillus plantarum, Lactobacillus helveticus, Streptococcus cremoris, Streptococcus thermophiles, Streptococcus intemedius, Streptococcus lactis, Streptococcus diacetilactis, Enterococcus feacalis, Lactococcus spp, Lactococcus lactis subsp., Pediococcus spp., Pediococcus cerevisiae, Pediococcus acidilactici, Pediococcus pentosaceus, Bifidobacterium spp. Bifidobacterium animalis, Bifidobacterium adolecentis, Bifidobacterium bifidum, Bifidobacterium breve, Bifidobacterium infantis, Bifidobacterium longum, Leuconostoc spp. L salivarius, L. paracasei, L. gasseri, L. reuteri, B. Bifidum, B. longum, B. infantis, Lb. delbrueckii, Lb. plantarum, Lb. pentosus, Lb. brevis, P. damnosus, Lb. collinoides, Lb. pentosus, Pediococcus spp., Lb. buohneri, Leuconostoc mesenteroides, Pedococcus pentosaceus, Lb. casei, Lb. kefir, Lb. acidophilus, Lb. helveticus, Lb. casei, Lb. bulgaricus, Lb. lactis, Lb. plantarum, Lb. brevis, Acetobacter spp., Streptococcus genera, Streptococcus spp., Streptococcus lactis, S. thermophilus, S. durans, S. cremoris, Lactobacillus alimentarus, L. Alimentarus, L multaromicus, L sanfrancisco, Lactococcus lactis ssp,* S. *cerevisia, Lactobacillus sakei, Lactobacillus alimentarius, Lactobacillus paralimentarius, Lactobacillus paracasei, Lactobacillus buchneri, Enterococcus faecium, Enterococcus mundtii, Enterococcus faecelis, Enterococcus casseliflavus, Lactobacillus pentosus, Enterococcus faecium, Pediococcus pentosaceus, Lactobacillus farciminis, Pichia kudriavzevii, Lactobacillus farciminis, Lactobacillus casei, Lactobacillus alimentarius, Pichia kudriavzevii, Candida humilis, L.lactis subsp. cremoris, Lb.delbrueckii subsp. lactis, Lb.helveticus, Lb.casei, Lb.delbrueckii subsp., Leuc.mesenteroides subsp. cremoris, Lb. johsonii, Lb.kefirenofacies, Lb. curvatus, P. acidilactici, P.pentosaceus, Lb. alimentarius, Cpiscicola, Leuc.mesenteroides, P.acidilactici, P.cerevisiae, Lb.pentosus, P.acidilactici, T.halophilus, Lb.sanfransiscensis, Lb.farciminis, Lb.fermentum, Lb.amylovorus, Lb.reuteri, Lb.pontis, Lb.panis, Lb.alimentarius, W. cibaria, O.oeni, Lcoryniformis, Lcurvatus, Ljugurti, Ljensenii, Lbucheneri, Lcellobiosus, Lcoprophilus, Lhilgardii, Lleichmannii, Ldextranicum, P. acidilactici, P.pentosaceus, S. thermophilus, Llactis subsp. diacety lactis, Llactis subsp. hordniae, Lgarvieae, Lrafinolactis, v.fuvialis, V.salmoninarum, Leuconostoc sp., Lcremoris, Ldextranicum, Lmesenteroides, Lparamesenteroides, Lgelidum, Lmmosum, Carnobacterium sp., Cdivergens, Cmobile, Cgallinarum, Cpiscicola, Vagococcus sp., v.fuvialis, V.salmoninarum, Lgarvieae, Lactococcus diacetylactis, Propionibacterium freudenreichii, Pediococcus sp, S.uvarum, Lb.coryniformis, Candida crusei, Weissella confusa, Hansenula silvicola, Debaryomyces hansenii, Trichosporon beigelli, Bacillus amyloliquefaciens, Torulopsis sp., Candida mycoderma, Lb.buchneri, P.acidilactici, Pediococcus pentasaceus, Lcellobiosus, EmundtiilEgallinarum, E casselifiavus, P.urinae-equi, Lb.murinus, Candida milleri, Eburtonii, E fibulinger, Issatchenkia orientalis, Candida pelliculosa, Ctropicalis, Pediococcus acidilactici, Pseudoplantarum, Pediococcus acidilactici, Pediococcus pentosaceus, Leuconostoc pseudomesenteroides, Weissella cibaria, Lb. paraplantarum, Issatchenkia orientalis, Candida glabrata, Pediococcus acidilactici, Kluyveromyces marxianu, Pichia kudriavzevi, Saccharomyces servazzi, Torulaspora delbrueckii, Kazachstania unispora, Saccharomyces barnettii.*

10. Microcapsule chargée en probiotique qui est protectrice contre la formation de moisissures et de mycotoxines, produite par le méthode selon la Revendication 1.

11. Utilisation de la microcapsule chargée en probiotiques selon la Revendication 10 en agriculture.

12. Utilisation de la microcapsule chargée en probiotiques selon la Revendication 11 avec des produits à base de raisin, de maïs, d'amande, de noisette, de dérivés d'arachide, de noix, de fruits/légumes frais/secs ou de produits céréaliers.

13. Utilisation de la microcapsule chargée en probiotiques selon la Revendication 10 avec des produits agricoles stockés.

14. Utilisation de la microcapsule chargée en probiotiques selon la Revendication 10 avec des produits alimentaires choisis parmi les fromages, les olives, les yaourts, les produits carnés, les fruits et les légumes ou des produits cosmétiques choisis parmi les produits de toilette, les crèmes, les gels et les émulsions
